# EUROPEAN PATENT SPECIFICATION

(11) **EP 1 996 603 B1**
(45) Date of publication and mention of the grant of the patent: **15.07.2009**
(21) Application number: 07711956.8
(22) Date of filing: 15.03.2007
(51) Int. Cl.: C07H 15/04, A61P 29/00, A61K 31/7048

(54) **LIPID A ANTAGONISTS WITH ANTI-SEPTIC SHOCK, ANTI-INFLAMMATORY, ANTI-ISCHEMIA AND ANALGESIC ACTIVITY**
LIPID-A-ANTAGONISTEN MIT ANTI-SEPTISCHER-SCHOCK-WIRKUNG, ENTZÜNDUNGSHEMMENDER WIRKUNG, ANTIISCHÄMISCHER WIRKUNG UND ANALGETISCHER WIRKUNG
ANTAGONISTES DES LIPIDES A PRÉSENTANT UNE ACTIVITÉ ANTI-CHOC SEPTIQUE, ANTI-INFLAMMATOIRE, ANTI-ISCHÉMIE ET ANALGÉSIQUE

(30) Priority: 22.03.2006 IT MI20060530
(43) Date of publication of application: 03.12.2008
(73) Proprietor: Università Degli Studi Di Milano - Bicocca, 20126 Milano (IT)
(72) Inventor: PERI, Francesco, I-20141 MILANO (IT); NICOTRA, Francesco Maria, I-20135 Milano (IT); COSTA, Barbara, Simona, 20092 Cinisello Balsamo (IT); GRANUCCI, Francesca, 20124 Milano (IT)
(74) Representative: Bianchetti, Giuseppe
(86) International application number: PCT/EP2007/002279
(87) International publication number: WO 2007/107285

(56) References cited:
- US-A1- 2006 040 891
- T. D. INCH AND G. J. LEWIS: "Use of Carbohydrate Derivatives for Studies of Phosphorus Stereochemistry" CARBOHYDRATE RESEARCH, vol. 45, 1975, pages 65-72, XP002438164
- F. PERI ET AL: "Synthesis and biological evaluation of novel lipid A antagonists" BIOORGANIC & MEDICINAL CHEMISTRY, vol. 14, 2006, pages 190-199, XP002438165
- A. V. DEMCHENKO ET AL: "Synthesis and Biological Evaluation of Rhizobium sin-1 Lipid A Derivatives" JOURNAL OF THE AMERICAN CHEMICAL SOCIETY, vol. 125, 2003, pages 6103-6112, XP002438396 cited in the application
- B. L. RAY ET AL: "The Biosynthesis of Gram-negative Endotoxin" THE JOURNAL OF BIOLOGICAL CHEMISTRY, vol. 259, 1984, pages 4852-4859, XP002438397 cited in the application
- A. ZAMYATINA ET AL: "Synthesis and purity assessment of tetra- and pentaacyl lipid A of Chlamydia containing (R)-3-hydroxyicosanoic acid" TETRAHEDRON, vol. 60, 2004, pages 12113-12137, XP002438398

## Description

### Field of the invention

The present invention relates to compounds able to inhibit the toxic effect of lipid A, the inflammatory activity of λ-carrageenan and to prevent pathologies mediated by the TLR-4 receptor.

### Background of the invention

Sepsis and septic shock are serious clinical syndromes related to high mortality rates caused by an uncontrolled systemic inflammatory response to bacterial lipopolysaccharides (LPS) in the blood of affected patients. LPS are components of the cell wall of Gram-negative bacteria, consisting of a hydrophilic oligosaccharide chain covalently linked to a lipo-disaccharide called lipid A. Lipid A is the membrane-anchoring moiety of LPS and is deemed to be the biologically active portion (toxic principle) of LPS.

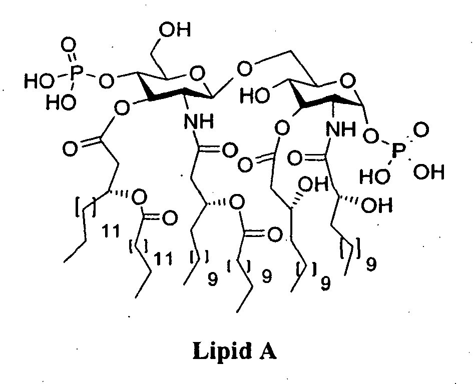

Lipid A triggers the production of a number of endogenous pro-inflammatory molecules, mainly cytokines (in particular TNFα) and chemokines, which are important mediators of the innate immunity. The reaction cascade leading to the production of these mediators starts with the formation of a complex between LPS and a plasma protein, the LPS-Binding Protein (LBP); thereafter, the LPS:LBP complex binds to the CD14 protein, which is both free and bound to the membrane, to form a CD14:LBP:LPS trimolecular complex. At this stage the Toll-like Receptor 4 (TLR-4) associates to the trimolecular CD14:LBP:LPS complex and the further binding to the MD-2 protein triggers the signal cascade that leads to the activation of the NFκB transcription factor and to the expression of cytokines and chemokine genes. Moreover, TLR-4 activation elicits the production of glutamate, prostaglandins and nitric oxide from glial cells. These agents are then capable of further enhancing glial activation and production of inflammatory mediators which sensitize dorsal horn neurones, thereby contributing to neuropathic pain. The key role of TLR-4 in microglia activation and consequently in the aetiology of neuropathic pain has been very recently demonstrated [F. Y. Tanga et al., Proc. Natl. Acad. Sci 2005, 102, 5856-5861]. Moreover, TLR-4-mediated inflammatory reactions play an important role in cerebral ischemia-reperfusion injury [C.X. Cao et al., Biochem. Biophys. Res. Commun. 2007, 353, 509-514].

Due to its role in the molecular mechanism described above, remarkable attention has been devoted to lipid A in pharmacology, since it is one of the known substances with the highest pro-inflammatory activity. Lipid As of different bacterial origins, or their synthetic agonists inducing co-stimulating activity have been used for some time in admixture with antigen proteins (or other synthetic antigens with sugar structure) in order to increase the immunogenicity of the latter for the development of vaccines. The former are known as adjuvants and are necessary to induce the production of antibodies with a sufficiently high titer and directed against the concerned antigen. On the contrary, synthetic compounds able to inhibit LPS and lipid A (antagonists) have been used as lead compounds for the development of drugs against septic shock.

The chemical structure of lipid As of diverse bacterial origin is different in the number of ramifications and unsaturations in the lipophilic chains. However, systematic studies evidenced that some structural features are common to all lipid A variants [Rietschel, E.T. et al.. The FASEB J. 1994, 8, 217-225]. According to their three-dimensional structure, lipid As interact in different ways with TLR-4. The essential structure responsible for the inflammatory and endotoxic effect of lipid A comprises a GlcNAcβ (1-6)GlcNAc disaccharide, two phosphoric esters at the C-1 and C-4' positions and an appropriate number of lipophilic chains bound to the C-2, C-3, C-2' and C-3' positions. On the basis of this structure-activity relationship synthetic lipid A analogues which proved active as agonists or antagonists were prepared. These compounds share a disaccharide structure, with the sole exception of compound ER-112022 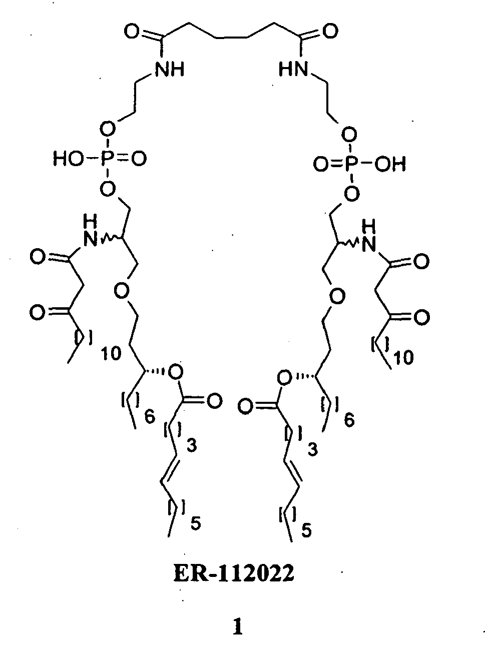 which is a phospholipide dimer wherein the two phosphate groups are linked by a linear spacer presumably endowed with high conformational mobility. Even if this compound lacks a disaccharide nucleus, it possesses a strong pro-inflammatory activity, similar to that of lipid A. The biological activity of lipid A was also correlated to its tridimensional form. Such form is mainly determined by the number, length and arrangement of the lipophilic chains, as well as by the number and distribution of the negative charges on the phosphate groups. Lipid As with an asymmetric distribution of the chains (4+2 type) have a tridimensional conical-trunk shape and have inflammatory activity (agonists), while the variants with a symmetric distribution (2+2 type) have a cylindrical shape and antagonist properties. Lipid As with conical shape interact with TLR-4 and induce a conformational change that activates signal transmission within the cells, while lipid As with cylindrical shape bind to the same receptor without inducing any conformational change and therefore without triggering any signal. Finally, lipid As with a slightly conical intermediate shape, due to a (3+2) arrangement of the chains, such as lipid A from *P. gingivalis*, have a weak pro-inflammatory activity due to activation of another TLR (TLR-2) and in some cases, for example in the case of lipid A from *R. sphaeroides* and *R. capsulatus*, have antagonist properties. These lipid A variants, commonly referred to as non-toxic lipid A variants, are devoid of pro-inflammatory activity and are able to inhibit lipid A from *E. coli in vitro* and *in vivo*. The chemical structure of both compounds led to the design of lipid A antagonists with a (2+2) symmetrical chain arrangement, among them compound E5564 (2) 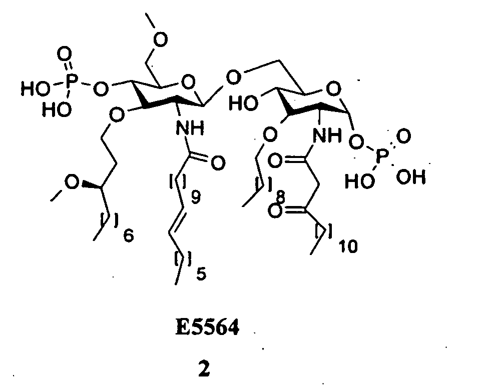 which showed a potent antagonist activity and is currently in clinical phase. Recently, it has been found that a synthetic analogue of lipid A from the nitrogen-fixing bacterium *R. sin-1,* of formula (3) 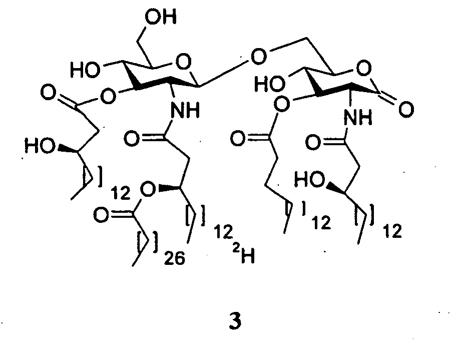 is able to antagonize the effect of lipid A from *E. coli* in *in vitro* experiments on human cell lines [A.V. Demchenko et al., J. Am. Chem. Soc. 2003, 125, 6103-6112]. It is worth noting that the disaccharide present in the structure of this compound has unique features, i.e. it does not contain phosphate groups and it contains the 2-aminogluconolactone monosaccharide moiety. Finally, a recent publication reported that a disaccharide with an unnatural N(OMe) β-glycosidic bond and having a (2+2) symmetric arrangement of the C14 lipophilic chains bound through ether bonds to the hydroxy groups of the Glc-Glc disaccharide was able to inhibit in a dose dependent way the inflammatory action of lipid A from *E. coli* on murine macrophages of the MT2 cell line [F. Peri et al., Bioorg. Med. Chem. 2006, 14(1), 190-199].

Lipid X, a monosaccharide precursor of lipid A, had been reported to antagonize the cellular effect of lipid A (B.L. Ray et al. J. Biol. Chem. 1984, 259, 4852-4859). However, a subsequent publication reported that the observed activity was not due to lipid X in the monosaccharide form, but to disaccharide traces deriving from its spontaneous condensation [H. Aschauer et al. J. Biol. Chem. 1990, 265, 9159- 9164].

Very few compounds are known that lack the disaccharide structure maintaining activities as lipid A agonists or antagonists. Monosaccharide-type lipid A analogues, among which compound GLA-58, despite the absence of the second, non-reducing monosaccharide moiety, preserved LPS-mimetic activities [R. Tamai, Y. Asai, M. Hashimoto, K. Fukase, S. Kusumoto, H. Ispida, M. Kiso, T. Ogawa, Immunology 2003, 110, 66-72].

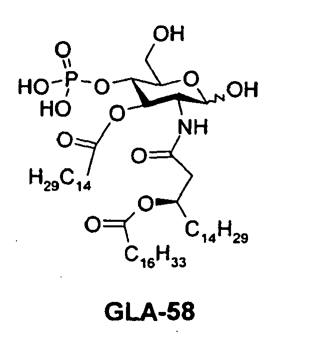

A panel of reducing N-acylated glucosylamines bearing a phosphate group in C4 and linear or branched 3-hydroxytetradecanoic acid ester and amide chains on C2 and C3, were recognized by murine macrophages both as LPS agonists and antagonist. Furthermore, compound GLA-58 showed to be active as LPS antagonists in human cells.

It has also recently been reported that [R.F. Tsuji et al. Clin. Exp. Allergy 2003, 33, 249-258] poly-galactans known as carrageenans (κ, 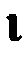 ,λ) which are able to induce a non-specific inflammation in men and animals, induce cytokines production in macrophages acting on TLR-4, the same receptor involved in the inflammatory pathway of LPS and lipid A. Substances able to inhibit the inflammatory and toxic action of lipid A are therefore also potential inhibitors of the inflammatory action of carrageenans.

### Description of the invention

The present invention relates to compounds of general formula (**I**) 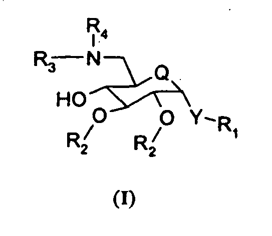 wherein:
Q represents oxygen;
Y represents oxygen;
R₁ is selected from hydrogen, C₁-C₁₀ alkyl and phenyl;
R₂, are both C₁₄ alkyl chains;
R₃ is tetrahydrofuran-1-yl or cyclopentyl;
R₄ is selected from hydrogen, C₁-C₁₀ alkyl or a R₄'X group wherein
R₄' is C₁-C₁₀ alkyl and X is an oxygen or sulphur atom;
and physiologically acceptable acid salts or ammonium quaternary salts thereof.

Particularly preferred are also the following compounds: methyl 6-deoxy-6-[*N*-(1'-tetrahydrofuranyl)-*N*'-methoxyamino)-2,3-di-*O-*tetradecyl-α-D-glucopyranoside **(Ia)** 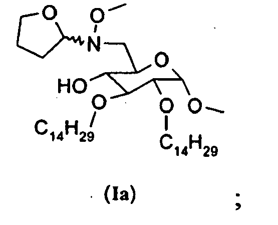 methyl 6-deoxy-6-[*N*-(cyclopentyl)-*N*'-methoxyamino)-2,3-di-*O*-tetradecyl-α-D-glucopyranoside (Ib) 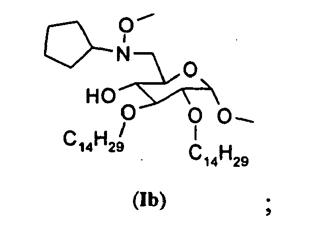 methyl 6-deoxy-6-cyclopentylamino-2,3-di-*O*-tetradecyl-α-D-glucopyranoside **(Ic)** 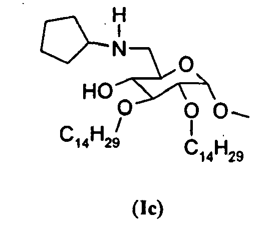 methyl 6-deoxy-6-*N,N',N"*-dimethylcyclopentylammonium-2,3-di-*O-*tetradecyl-α-D-glucopyranoside **(Id)**

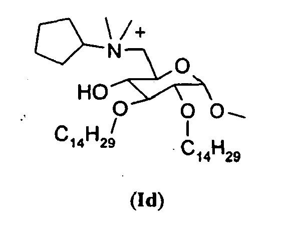

These compounds can be prepared as illustrated in the following scheme 1, by conversion of the commercially available methyl α-D-glucopyranoside to methyl 4,6-*O*-(4-methoxybenzylidenee)-α-D-glucopyranoside **4** (94% yield) by treatment with anisaldehyde dimethylacetal (ADMA) and camphorsulfonic acid (CSA). Alkylation of **4** with tetradecyl bromide in the presence of NaH gives **5** (74% yield), whose benzylidenee ring is regioselectively opened by treating with LiAlH₄ and AlCl₃, thus obtaining **6** (86% yield). The oxidation with Dess-Martin periodinane of the free hydroxyl group on C6 of **6** gives the corresponding aldehyde **7** that can be directly converted without isolation into **8** by reductive amination using cyclopentylamine and NaBH₃CN (75% yield over two steps). Removal of the C4 *p*-methoxybenzyl (PMB) group from **8** in acidic conditions (TFA) gives (**Ic**) (80% yield), which can be converted into the respective quaternary ammonium salt by reaction with an alkyl iodide; treatment with methyl iodide in the presence of sodium carbonate affords compound (**Id**) in 94% yield. Alternatively, aldehyde **7** can be reacted with *O*-methylhydroxylamine hydrochloride in pyridine affording the corresponding C6 *O*-methyl oxime with a yield of 65% over two steps. The methyloxime is then reduced to methyl hydroxylamine **9** by treatment with sodium cyanoborohydride in glacial acetic acid (92% yield). The PMB ether group can be cleaved with TFA/CH₂Cl₂ affording **10** (yield 90%), which is finally reacted with dihydrofuran (DHF) in the presence of the mild acid catalyst pyridinium *p-*toluensulfonate (PPTS) to give (**Ia**) in 65% yield. Alternatively, compound **10** can be reacted with bromocyclopentane in the presence of a base like diisopropylethylamine (DIPEA) to obtain compound (**Ib**).

Compound (**Ia**) is chemically unstable due to the presence of the *N,O* acetal group at C6 and is obtained as a mixture of two diastereoisomers with opposite stereochemistry of the carbon atom at the α position of the tetrahydrofuran ring. Compound (**Ib**) is more stable than compound (**Ia**) and is obtained in the form of a pure isomer. 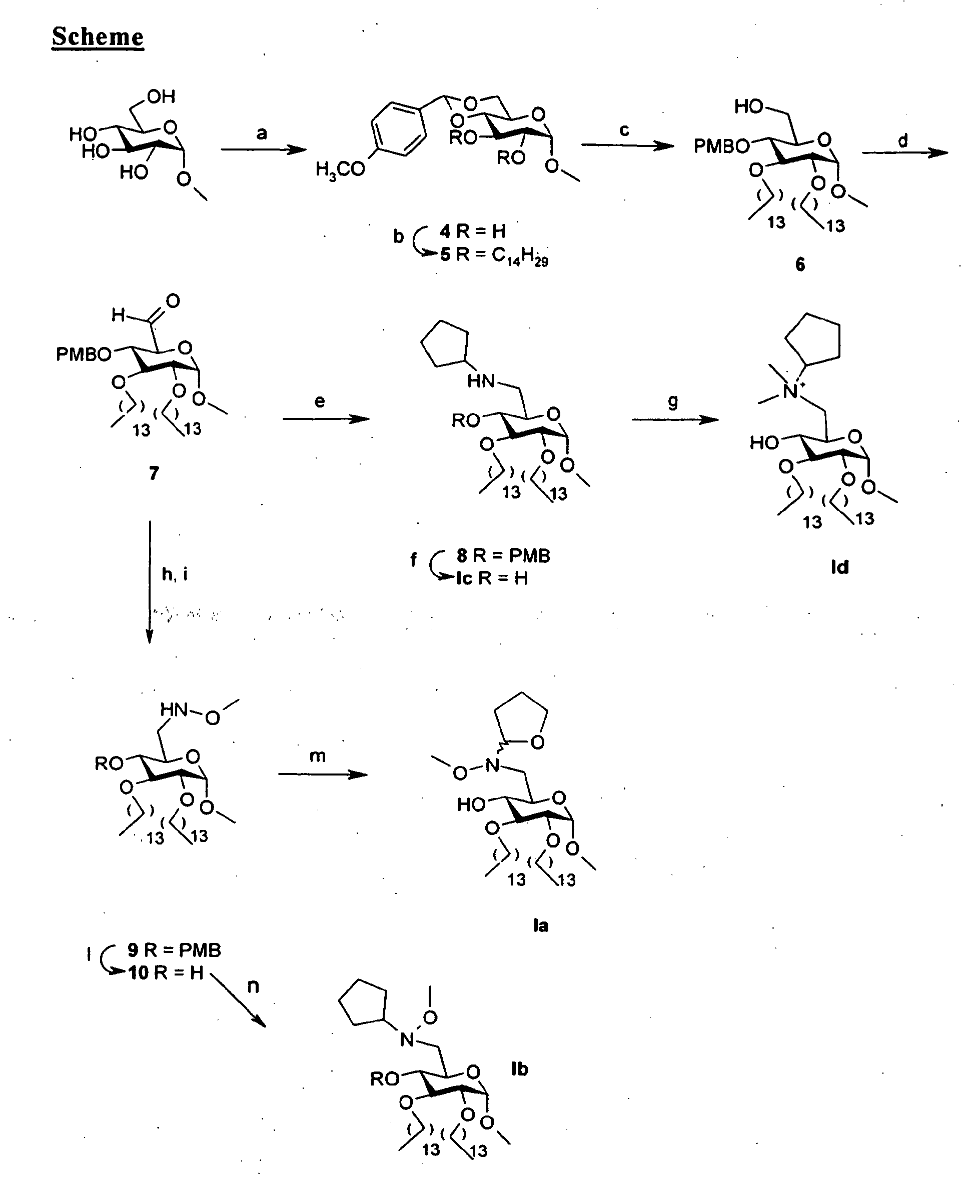 Scheme 1. Reagents and conditions: a) ADMA, CSA, DMF, 94%; b) C₁₄H₂₉Br, NaH, DMF, 74%; c) LiAlH₄, AlCl₃, CH₂Cl₂/Et₂O, 86%; d) Dess- Martin periodinane, CH₂Cl₂; e) cyclopentylamine, NaBH₃CN, AcOH, CH₂Cl₂, MeOH, 75% over two steps; f) TFA, CH₂Cl₂, 80%;g) CH₃I, Na₂CO₃, DMF, 94%; h) *O*-methylhydroxylamine hydrochloride, pyridine, 65% from 6, i) NaBH₃CN, glacial AcOH, 92%; l) TFA, CH₂Cl₂, 90%, m) DHF, PPTS, CH₂Cl₂, 65%; n) bromocyclopentane, DIPEA.

Other compounds of formula (**I**) can be obtained in a similar way starting from glucopiranose by suitable fuctionalisation and protection-deprotection reactions, which are well known to the skilled chemist.

The compounds formula (**I**) behave as lipid A and λ-carrageenans inhibitors and proved able to exert anti-inflammatory, anti-ischemic and analgesic effects. Therefore, the compounds of the invention can be used for the preparation of pharmaceutical compositions for the treatment of inflammatory states, ischemia and pain, in particular neuropathic pain. Such compositions can be prepared with conventional techniques and excipients, such as those disclosed in Remington's Pharmaceutical Sciences Handbook, XVII ed. Mack Pub., N.Y., U.S.A.

The invention will be now illustrated in greater detail in the following experimental section.

### Description of the figures

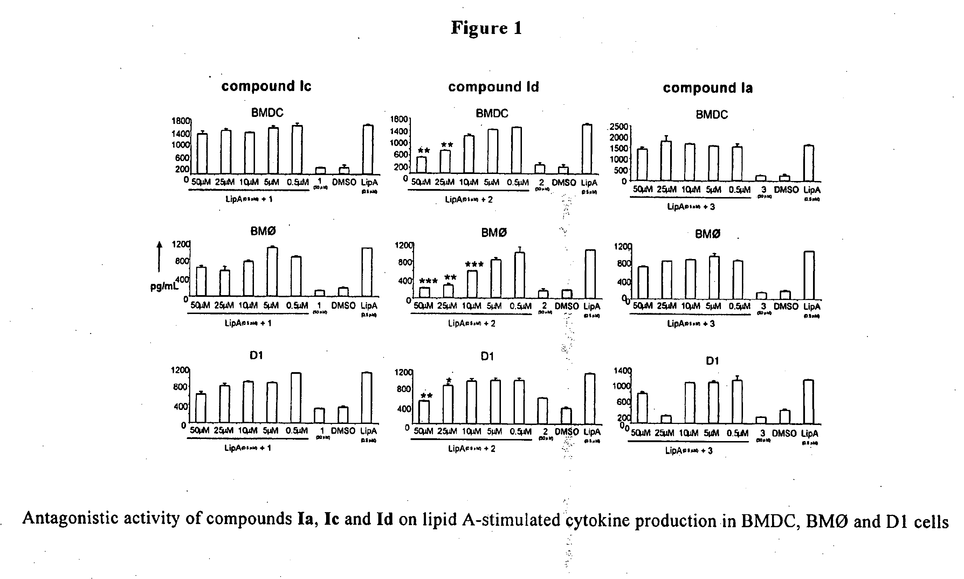

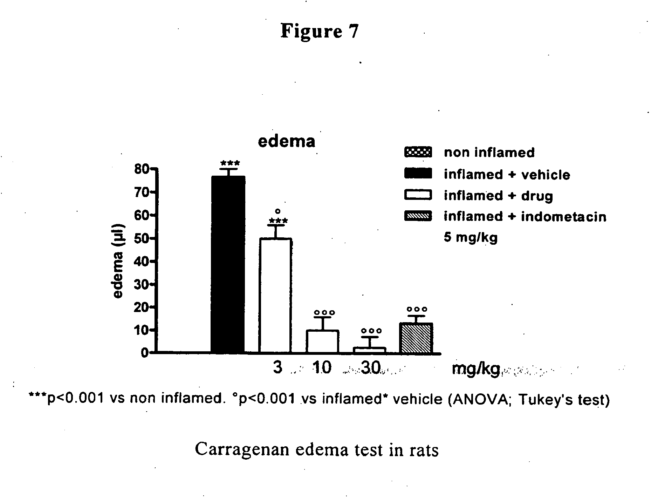

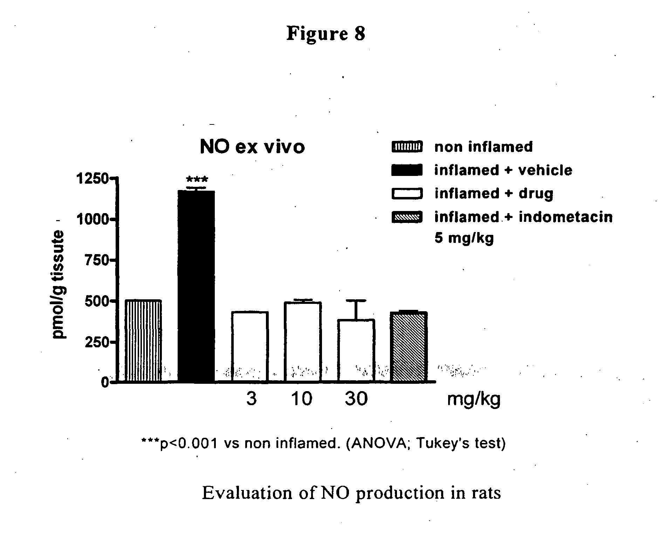

**Figure 1**. Antagonistic activity of compounds **Ia, Ic** and **Id** on lipid A-stimulated cytokine production in BMDC, BMØ and D1 cells. TNFα production was measured in the supernatants 24 h later. From the right: lipid A (0.5µM), cells treated with lipid A alone; DMSO: cells incubated in the presence of complete medium plus DMSO; inhibitor 50 µM, cells treated only with monosaccharides **Ia, Ic** and **Id;** other columns, lipid A + compounds **Ia, Ib** and **Ic** at increasing doses. The data represent means and standard deviations of triplicate wells. This is a representative of three independent experiments. *, p < 0.05; **, p < 0.01; ***, p< 0.001.

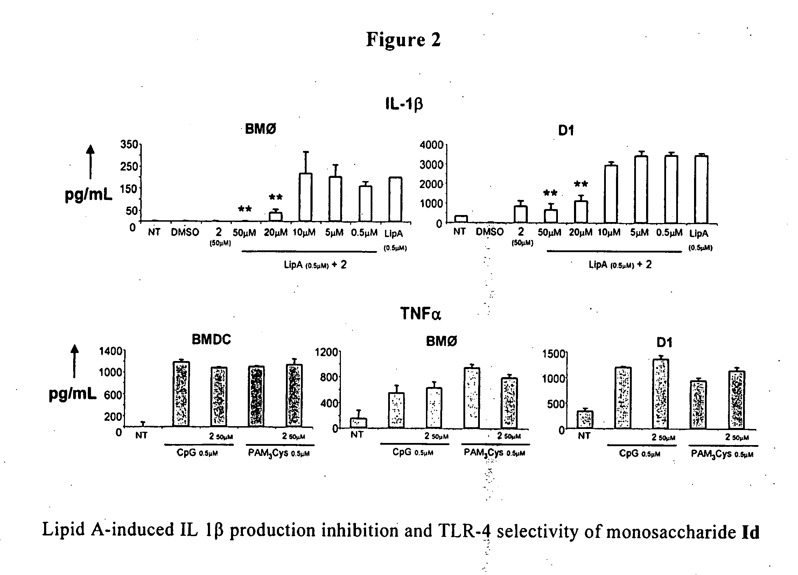

**Figure 2****.** TLR-4 selectivity of monosaccharide **Id.** Upper panels, the antagonistic activity of compound **Id** was investigated by testing its ability to interfere with IL-1β production 24 h after lipid A stimulation. IL-1β was measured in the supernatants by ELISA. Lipid A (0.5µM), cells treated with lipid A alone; NT: cells incubated in the presence of complete medium; DMSO: cells incubated in the presence of complete medium plus DMSO; inhibitor 50 µM, cells treated only with monosaccharide **Id;** other columns: lipid A + compounds **Id** at increasing doses. The data represent means and standard deviations of triplicate wells. This is a representative of three independent experiments. Lower panels, TNFα production measured by ELISA 24 h after stimulation with CpG (selective for TLR-9) or PAM₃Cys-SK₄ (selective for TLR-2) in the presence of compound **Id.** NT: cells incubated in the presence of complete medium plus DMSO; other columns: cells treated with CpG or Pam₃Cys-SK₄ in the presence or absence of compound **Id** at the indicated concentration. *, p < 0.05; **, p < 0.01; ***, p< 0.001

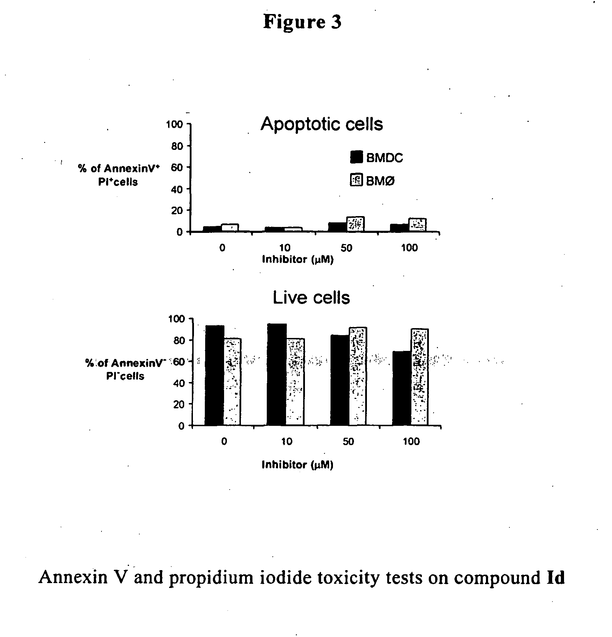

**Figure 3****.** Compound **Id** does not induce death of MΦ and DC. Mouse DC and MΦ were either left untreated or treated for 24 h with compound **Id** at the indicated concentrations. The cells were then analyzed by FACS for the presence of annexin V and propidium iodide (PI) double positive cells. Upper panel, percent of annexin V and PI double positive cells. Lower panel, percent of annexin V and PI double negative cells.

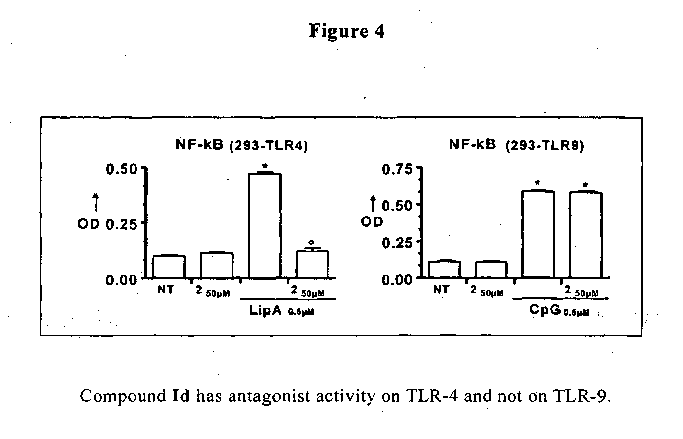

**Figure 4****.** Selective antagonistic activity of **Id** on TLR-4 receptor. HEK-293 cells stably transfected with human TLR-4A (left) or TLR9 (right) gene were treated respectively with lipid A alone or lipid A in the presence of monosaccharide **Id** or with CpG alone or CpG in the presence of monosaccharide **Id**. NF-kB activation was evaluated in the nuclear extract 2 h later and expressed as optical density (OD) at 450 nm. NT: non treated cells. The data represent means and standard errors of duplicate wells. This is a representative of three independent experiments. *p<0.001 vs NT cells; °p<0.001 vs Lipid A alone (ANOVA; Tukey's test).

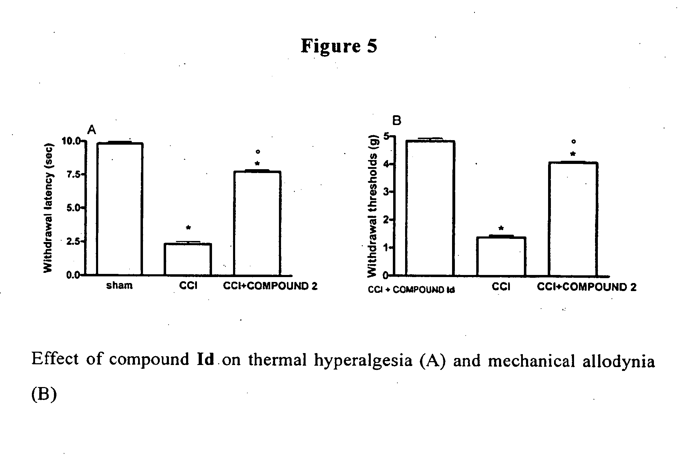

**Figure 5****.** Effect of compound **Id** daily administered in CCl mice for 1 week from the day after surgery on thermal hyperalgesia (A) and mechanical allodynia (B). *P ≤ 0.001 vs CCl (ANOVA, Tukey's test).

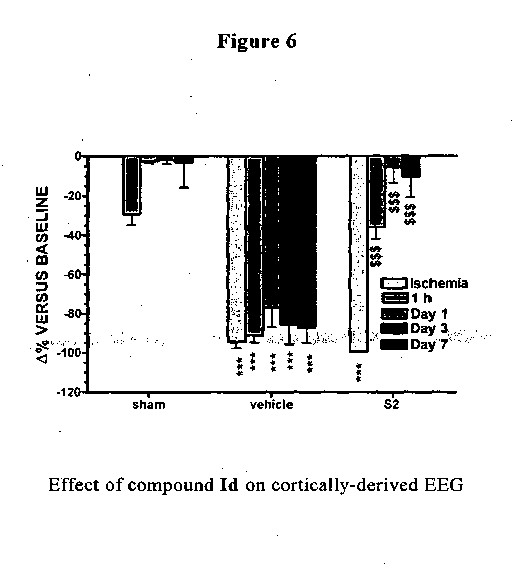

**Figure 6****.** Effect of compound **Id** (10 mg/kg), given i.p., 30 min before bilateral carotid occlusion, on cortically derived EEG mean total spectral power evaluated as the difference (Δ%) from the pre-ischemic value in gerbils. *** P < 0.001 as compared with sham group, same time; $$$ P < 0.001 as compared with vehicle group, same time (2-way ANOVA followed by Bonferroni's test).
**Figure 7****.** Effect of different doses of compound **Id** on carrageenan induced edema in mice measured 4h after the exposure and expressed as difference between the paw volume of treated paw versus the contralateral paw. Indomethacin (non steroidal anti-inflammatory drug) treatment represents the positive control.
**Figure 8**. Effect of treatment with compound **Id** in mice on nitric oxide production measured in paw homogenate 4h after the exposure and expressed as content of nitrite/nitrate, the end products of nitric oxide metabolism.

### EXPERIMENTAL SECTION

### Chemistry

### General procedures

All solvents were dried over molecular sieves (4 A, Fluka) for at least 24 h before use. When dry conditions were required, the reactions were performed under argon atmosphere. Thin-layer chromatography (TLC) was performed on Silica Gel 60 F254 plates (Merck) with UV detection, or using a developing solution of conc. H₂SO₄/EtOH/H₂O (5:45:45), followed by heating at 180°C. Flash column chromatography was performed on silica gel 230-400 mesh (Merck). Mixtures of petroleum ether (boiling range 40-60°C) and ethyl acetate were used as eluent. ¹H and ¹³C NMR spectra were recorded on a Varian 400 MHz MERCURY instrument at 300 K. Chemical shifts are reported in ppm downfield from TMS as internal standard; carbon signals of linear C₁₄ chains on C-2 and C-3 have been omitted in the carbon spectra assignment.

Mass spectra were recorded on a Fourier Transform Ion Cyclotrone Resonance (FT-ICR) instrument (model APEXII, Bruker Daltonics), equipped with a 4.7 T Magnet (Magnex).

Optical rotations were measured at room temperature, using the sodium D line, on a P3002 electronic polarimeter (A. Krüss, Germany).

### Step a) Methyl 4,6-O-(4-methoxybenzylidene)-α-D-glucopyranoside (4)

A solution of methyl α-D-glucopyranoside (10 g, 51.5 mmol) in dimethylformamide (DMF, 50 ml) was added with a catalytic amount of camphorsulfonic acid (CSA) and anysaldehyde dimethylacetal (ADMA, 10 ml, 51.5 mmol) and the mixture was kept under magnetic stirring and low vacuum to remove the methanol formed during the condensation reaction. After 40 min the solvent was evaporated off. The residue was added with a NaHCO₃ saturated solution and the resulting diphasic mixture was kept under vigorous stirring for one hour. The resulting precipitate was filtered and washed with an ice-cold bicarbonate solution (100 ml). The residue was triturated in hexane to afford compound **4** as a white solid (15 g, 94%). **4:** *R_{f}* = 0,31 (EtOAc/MeOH/H₂O 7:2:1).

### Step b) Methyl 4,6-O-(4-methoxybenzylidene)-2,3-di-O-tetradecyl-α-D-glucopyranoside (5)

A solution of **4** (8 g, 25.6 mmol) in DMF (80 ml), was added under stirring with sodium hydride in portions (60% suspension in mineral oil, 6.4 g, 160 mmol). Thereafter, tetradecyl bromide (38 ml, 128 mmol) was added drop by drop and stirring was continued at 60°C for at least 12 hours, then the mixture was cooled and added with methanol (20 ml) and the resulting solution was kept under stirring for further 20 minutes, to hydrolyse the excess of sodium hydride. The solvents were evaporated off and the residue was dissolved in dichloromethane (500 ml), then added with an aqueous solution of citric acid (400 ml). After separation from the aqueous phase, the organic phase was dried over sodium sulphate, filtered and evaporated. After purification of the residue by flash chromatography (eluent: 85:15 petroleum ether/AcOEt) compound **5** was obtained as a colourless oily solid. (13,3 g, 74%). 5: *R_{f}* = 0.32 (9:1 petroleum ether/AcOEt); [α]_{D} = +23° (c=1 in CHCl₃); ¹H-NMR (400 MHz, CDCl₃): δ (ppm) = 7.40-6.85 (A₂X₂q, 4H, aromatic), 5.48 (s, 1H, OCH₃), 4.77 (d, *J*=3.7 Hz, H-1), 4.24 (dd, *J*=9.7, 4.4 Hz, 1H, H-6a), 3.80 (s, 3H, OCH₃), 3.77-3.60 (m, 8H), 3.47 (t, *J* = 9.3 Hz, 1H), 3.42 (s, 3H, OCH₃), 3.34 (dd, *J*=9.3, 3.7 Hz, 1H, H-2), 1.5-1.6 (m, 4H, H-β), 1.22 (bs, 44H, CH₂), 0.85 (t, J=5.8 Hz, 6H, CH₃). ¹³C-NMR (400 MHz, CDCl₃): δ (ppm) = 161.2 (Cₐᵣ), 127.4 (Cₐᵣ), 128.4 (Cₐᵣ), 114.0(Cₐᵣ), 105.1, 98.4 (C-1), 83.4, 80.8, 74.5 (OCH₂), 73.5 (OCH₂), 71.6 (OCH₂), 71.2, 70.2 (C-6), 69.6, 57.7 (OCH₃), 55.6 (OCH₃), 32.4, 30.8, 30.5, 30.1, 30.1, 30.0, 29.9, 29.8, 26.4, 23.1, 14.6. MS (MALDI-TOF): *m*/*z*: 705.6 [M+H]⁺, 727.5 [M+Na]⁺.

### Step c) Methyl 4-O-(4-methoxybenzyl)-2,3-di-O-tetradecyl-α-D-glucopyranoside (6)

Compound **5** (1.0 g, 1.41 mmol) was dissolved in 2:1 Et₂O/CH₂Cl₂ (70 ml) under argon atmosphere, then added drop by drop with LiAlH₄ (1M in THF, 7.2 ml) and AlCl₃ (1.16 g, 8.72 mmol) in ethyl ether (25 ml) and the resulting mixture was stirred under reflux for 4 hours. After cooling the mixture to room temperature, the residue was diluted with AcOEt (300 ml) and water (300 ml). The organic phase was separated from the aqueous one, washed with brine (3 x 200 ml), dried over sodium sulphate, filtered and evaporated. The residue was purified through flash chromatography (eluent: 7:3 petroleum ether/AcOEt) to obtain pure compound **6** (860 mg, 86%). 6: *R_{f}*=0.25 (8.5:1.5 petroleum ether/AcOEt). [α]_{D} = +46° (c=1 in CHCl₃); ¹H-NMR (400 MHz, CDCl₃): δ (ppm) = 7.36 (m, 2H, aromatic), 6.92 (m, 2H, aromatic), 4.86-4.57 (ABq, 2H, *J*=10.6Hz, C*H₂*-(4-*O*MePh)), 4.75 (d,1H, *J*=3.5 Hz, H-1), 3.90 - 3.54 (m, 11H, H-3, H-5, H-6a,H-6b, H-α), 3.41 (dd, 1H, *J*= 9.8, 9.0 Hz, H-4), 3.37 (s, 3H, OCH₃), 3.27 (dd, 1H, *J*=3.6, 9.7 Hz, H-2), 1.5-1.6 (m, 4H, H-β), 1.22 (bs, 44H, CH₂), 0.85 (t, 6H, *J*=5.8 Hz, CH₃). ¹³C-NMR (400 MHz, CCl₃): δ (ppm) = 159.2 (Cₐᵣ), 130.2 (Cₐᵣ), 129.47 (Cₐᵣ), 113.9(Cₐᵣ), 98.3 (C-1), 81.3, 80.7, 73.9 (OCH₂), 73.5 (OCH₂), 71.6 (OCH₂), 71.1, 70.2 (C-6), 69.6 55.6 (OCH₃), 55.6 (OCH₃), 32.4, 30.8, 30.5, 30.1, 30.1, 30.1, 30.0, 29.9, 29.8, 26.4, 23.2, 14.6 (CH₂). MS (MALDI-TOF): *m*/*z*: 707.4 [M+H]⁺, 729.4 [M+Na]⁺, 745.3 [M+K]⁺.

### Step d) Methyl 4-O-(4-methoxybenzyl)-2,3-di-O-tetradecyl-α-D-glucohexodialdo-1,5-pyranoside (7)

A solution of compound **6** (800 mg, 1.13 mmol) in anhydrous CH₂Cl₂ (35 ml) was added with Dess-Martin periodinane (720 mg, 1.70 mmol) under argon atmosphere. After one hour the crude product was diluted with dichloromethane (200 ml) and added with a 1/1 (v/v) NaHCO₃/Na₂S₂O₃ (200 ml) saturated solution. After vigorous stirring, the two phases were separated and the organic one was washed with water (200 ml), then dried with sodium sulphate and the residue was evaporated off. The oily residue (aldehyde) was used as such without purification for the following reaction. **7**: *R_{f}* = 0,30 (petroleum/AcOEt 7.5:2.5).

### Spep e) Methyl 6-deoxy-6-cyclopentylamino-4-O-(4'-methoxybenzyl)-2,3-di-O-tetradecyl-α-D-glucopyranoside (8)

A stirred solution of aldehyde **7** (1.690 g, 2.4 mmol), cyclopentylamine (970 µL, 9.7 mmol), NaCNBH₃ (640 mg, 9.7 mmol) and AcOH (500 µL) in dry CH₂Cl₂/MeOH (2:1 v/v, 30 mL), was warmed at 60°C for 2 h under argon atmosphere; after this time TLC analysis (7:3 toluene/EtOAc) revealed that the reaction was complete. The solvents were then evaporated *in vacuo*, the residue dissolved in CH₂Cl₂ (50 mL) and washed with saturated aqueous bicarbonate and brine. The organic phase was dried over sodium sulphate, filtered and the solvent was evaporated off. The residue was purified by flash column chromatography on silica gel (9.5:0.5 EtOAc/MeOH) affording **8** (1.392 g, 75% yield) as a pale yellow powder. ¹H-NMR (CDCl₃) δ= 0.95 (m, 6H), 1.21 (m, 44H), 1.40-1.60 (m, 12 H), 2.62 (dd, 1H, *J* = 12.1, 6.8 Hz), 2.84 (dd, 1H, *J* =12.1, 2.8 Hz), 3.00 (quintet, 1H, *J* =6.8 Hz), 3.26 (dd, 1H, *J* =9.6, 3.4. Hz), 3.31 (t, 1H, *J*= 9.3 Hz), 3.37 (s, 3H), 3.50-4.0 (m, 6H), 3.79 (s, 3H), 4.55-4.80 (ABq, 2H), 4.71 (d, 1H, *J*=3.5 Hz), 6.80-7.20 (A₂X₂, 4H). ¹³C-NMR (CDCl₃) δ= 14.6, 23.1, 24.4, 26.4, 26.7, 29.8, 29.9-30.1 (signals of linear C₁₄ chain CH₂), 31.0, 32.4, 49.5, 55.59, 55.64, 59.9, 69.6, 72.0, 74.0, 74.8, 79.4, 81.1, 81.9, 98.1, 114.0, 129.9, 130.8, 159.4. HRMS (FT-ICR): calcd for C₄₈H₈₈NO₆: 774.6612 [M+1]; found: 774,6604.

### Step h) Methyl 4-O-(4-methoxybenzyl)-2,3-di-O-tetradecyl-α-D-glucohexodialdo-1,5-pyranose-6-O-methyloxime

Aldehyde 7 (800 mg, 1.13 mmol) was dissolved in pyridine (12 ml) and added with *O*-methyl hydroxylamine hydrochloride (142 mg, 1.7 mmol). After 1.5 h, the solvent was evaporated off under vacuum and the residue was purified by flash chromatography (eluent: 9:1 petroleum ether/AcOEt), to obtain the title compound (540 mg, 65%). *R_{f}* =0.70 (7.5:2.5 petroleum ether/AcOEt); ¹H-NMR (400 MHz, CDCl₃): δ (ppm) = 7.17 (d, 1H, J=7.6 Hz, H-6), 7.13-6.77 (A₂X₂q, 4H, J=8.6 Hz, aromatic), 4.71 (d, 1H, J = 3.4 Hz, H-1), 4.65, 4.44 (ABq, 2H, *J*=10.6Hz, C*H₂*-(4-*O*MePh)), 4.10 (dd, 1H, J= 9.9, 6.3 Hz, H-3), 3.82 (s, 3H, OCH₃), 3.72 (s, 3H, OCH₃), 3.33 (s, 3H, OCH₃), 3.45-3.78 (m, 5H), 3.20-3.28 (m, 2H), 1.50-1.60 (m, 4H, H-β), 1.22 (bs, 44H, CH₂), 0.85 (t, 6H, J= 5.8 Hz, CH₃). ¹³C-NMR (400 MHz, CDCl₃): δ (ppm) = 159.4 (Cₐᵣ), 147.3 (Cₐᵣ), 130.3 (Cₐᵣ), 129.9 (Cₐᵣ), 113.9 (C6), 98.4 (Cl), 81.4, 80.4, 79.7, 76.0, 76.5, 72.1, 68.5, 62.1 (NOCH₃), 55.9 (OCH₃), 55.9 (OCH₃), 32.3, 31.0, 30.4, 30.1, 30.1, 30.1, 30.0, 30.0, 29.9, 29.8, 26.7, 26.4, 23.1, 14.6 (O(CH₂)₁₃*C*H₃). MALDI-TOF (DHB): m/z: 757.1 [M+Na]⁺, 773.3 [M+K]⁺.

### Step i) Methyl 6-methoxylamino-4-O-(4-methoxybenzyl)-2,3-di-O-tetradecyl-6-deoxy-α-D-glucopyranoside (9)

A solution of the compound from step h) (500 mg, 0.68 mmol) in glacial acetic acid (35 ml) was added with sodium cyanoborohydride (214 mg, 3.4 mmol) and the resulting solution was stirred for 4 hours. The solvent was then evaporated off and the residue was purified by flash chromatography (eluent: 8:2 petroleum ether/AcOEt) to obtain compound **9** (460 mg, 92%). **9**: *R_{f}* = 0.42 (7.5:2.5 petroleum ether/AcOEt); ¹H-NMR (400 MHz, CDCl₃): δ (ppm) = 7.25 (m, 2H, aromatic), 6.83 (m, 2H, aromatic), 5.60 (bs, 1H, NH), 4.82, 4.55 (ABq, 2H, *J*=10.6Hz, C*H₂*-(4-*O*MePh)), 4.72 (d, 1H, J=3.5 Hz, H-1), 3.90-3.55 (m, 5H, 4H-α, H-3), 3.80 (s, 3H, OCH₃), 3.49 (s, 3H, OCH₃), 3.38 (s, 3H, OCH₃), 3.30-3.20 (m, 4H, H-2, H-4, H-5, H-6a), 2.84 (dd, 1H, J=13.2, 7.7 Hz, H-6b). ¹³C-NMR (400 MHz, CDCl₃): δ (ppm) = 159.4, 130.7, 129.8, 114.5, 98.0, 97.9, 82.0, 81.3, 79.9, 74.9, 74.1, 72.1, 67.1, 61.6, 61.6, 55.6, 55.6, 55.4, 55.4, 52.7, 32.3, 31.0, 30.5, 30.1, 30.1, 29.9, 29.7, 26.46, 23.15, 14.6. MALDI-TOF: m/z: 758.1[M+Na]⁺, 774.1 [M+K]⁺.

### Step 1) Methyl 6-methoxylamino-2,3-di-O-tetradecyl-6-deoxy-α-D-glucopyranoside (10)

Compound **9** (200 mg, 0.27 mmol) was dissolved in trifluoroacetic acid (TFA), then added with CH₂Cl₂ (1/1, 20 ml) at 0°C and the resulting solution was stirred for one hour at this temperature. The solvents were evaporated off under vacuum and the residue was purified by flash chromatography (eluent 35% AcOEt in petroleum ether), thus obtaining pure compound **10** (150 mg, 90%). **10:** R_{f} =0.42 (7.5:2.5 petroleum ether/AcOEt); ¹H-NMR (400 MHz, CDCl₃): δ (ppm) = 4.76 (d, 1H, J = 3.5 Hz, H-1), 3.89 (m, 1H, H-α), 3.82 (m, 1H, H-5), 3.63 (m, 1H, H-α), 3.60-3.50 (m, 2, H-3, H-4), 3.54 (s, 1H, OCH₃), 3.32 (dd, 1H, J=3.1, 13.5 Hz, H-6a), 3.27 (dd, 1H. J=3.5, 9.5 Hz, H-2), 3.00 (dd, 1H, J=7.2, 13.5 Hz, H-6b), 1,5-1,6 (m, 4H, H-β), 1,22 (bs, 44H, CH₂), 0,85 (t, 6H, J= 5.8 Hz, CH₃). ¹³C-NMR (400 MHz, CDCl₃): δ (ppm) = 98.2 (C-1), 81.2, 80.9, 74.9, 73.4, 72.1, 67.3, 61.8, 55.5, 53.7, 32.3, 31.0, 30.5, 30.1, 30.1, 29.9, 29.7, 26.46, 23.15, 14.6. MALDI-TOF (DHB): m/z: 639.4 [M+Na]⁺, 655.1 [M+K]⁺.

### Step m) Methyl 6-deoxy-6-[N-(1'-tetrahydrofuranyl)-N'-methoxyamino]-2,3-di-O-tetradecyl-α-D-glucopyranoside (Ia)

To a solution of monosaccharide **10** (174 mg, 0,28 mmol) in dry CH₂Cl₂, pyridinium *p*-toluenesulfonate (7 mg, 0.028 mmol) and dihydrofuran (64 µL, 0.42 mmol) were added under argon atmosphere and the mixture was stirred at r.t. for 3h. After this time, the starting reagent was completely converted into the *N,O* bis-THF adduct as assessed by TLC analysis (toluene/EtOAc, 7:3). The solvent was evaporated off and the residue was dissolved in AcOH/THF/H₂O 4:4:1 (9 mL). The conversion of the *bis*-THF adduct into (**Ia**) was followed by TLC and was complete after 6 h. The solvents were then evaporated off *in vacuo*, and the residue was purified by flash column chromatography on silica gel (AcOEt/petroleum ether, gradient of polarity starting from 2.5: 7.5) affording (**Ia**) as a white powder (129 mg, 68% yield). ¹H-NMR (CDCl₃): δ (ppm) = 0.85 (t, 6H, *J* = 5.8 Hz), 1.22 (m, 44H), 1.50-1.60 (m, 4H), 1.72 (m, 2H), 1.91 (m, 2H), 2.78 (bt, 1H, H-6), 3.11 (bt, 1H, H-4), 3.23 (dd, 1H, *J* = 9.4, 3.6 Hz, H-6), 3.40 (s, 3H, NOCH₃), 3.50-3.70 (m, 3 H), 3.90 (bm, 2H, H-4'), 4.20 (bm, 1H, H-1'), 4.73 (d, 1H, *J* = 3.6 Hz, H-1). ¹³C-NMR (400 MHz, CDCl3): δ (ppm) = 97.5, 92.03, 80.65, 79.20, 76.42, 72.08, 70.84, 61.27, 59.92, 58.71, 53.89, 30.60, 29.0-28.0 (bulk of CH₂ signals), 21.39, 12.84. [α]²⁰_{D} = + 42.45° (c = 0.5, MeOH). HRMS (FT-ICR): calcd for C₄₀H₇₉NO₇: 685.5857; found: 708.5710 [M+Na]⁺. Elemental analysis calcd, (%) for C₄₀H₇₉NO₇: C 70.03, H 11.61, N 2.04; found: C 70.07, H 11.57, N 2.01.

### Step n) Methyl 6-deoxy-6-[N-(cyclopentyl)-N'-methoxyamino]-2,3-di-O-tetradecyl-α-D-glucopyranoside (Ib)

Compound **10** (25 mg, 0.04 mmol) was dissolved in anhydrous DMF (1 ml) under argon atmosphere and added with cyclopentylbromide (45 µL, 0.4 mmol) and diisopropylethylamine (DIPEA, 70 µL, 0,4 mmol). The mixture was stirred for 20 hours at 70°C, then the solvent was evaporated off under vacuum and the residue was diluted with dichloromethane (10 ml) and washed three times with brine. The organic phase was then dried over sodium sulphate, filtered and the solvent was evaporated off. The residue was purified by column flash chromatography, using 9/1 petroleum ether/ethyl acetate as eluent. Yield: 10%. ¹H-NMR (400 MHz, CDCl₃): δ (ppm) = 4.72 (d, 1H, J=3.5 Hz, H-1), 4.10 (dd, 1H, J=9.0.5.0 Hz), 3.81 (s, 3H, OCH₃), 3.70-3.50 (m, 7H), 3.44 (s, 3H, NOCH₃), 3.41 (t, 1H, J=8.7 Hz), 3.22 (dd, 1H, J=9.1, 3.5 Hz, H-2), 1.5-1.6 (m, 4H, H-β), 1.22 (bs, 44H, CH₂), 0,85 (t, 6H, J= 5.8 Hz, CH₃). Massa (ESI): m/z = 684.61 [M+H]⁺, 706.59 [M + Na]⁺.

### Step f) Methyl 6-deoxy-6-cyclopentylamino-2,3-di-O-tetradecyl-α-D-glucopyranoside (Ic)

Compound **8** (1.400 g, 1.9 mmol) was dissolved in TFA/CH₂Cl₂ (1:1 v/v, 20 mL) and stirred for 1 h. After this time, PMB hydrolysis was complete, as assessed by TLC analysis (EtOAc/MeOH, 9:1). The solvents were evaporated off in vacuo, the residue was dissolved in CH₂Cl₂ (50 mL) and washed with saturated aqueous bicarbonate and brine. The organic phase was dried over sodium sulphate, filtered and the solvent was evaporated off. The residue was purified by flash column chromatography on silica gel (9.5:0.5 EtOAc/MeOH) affording (**Ic**) (0.993 g, 80% yield) as a yellow powder. ¹H-NMR (CDCl₃) δ = 0.95 (m, 6H), 1.21 (m, 44H), 1.40-1.60 (m, 12 H), 2.59 (bs, 1H), 2.81 (dd, 1H, *J* =11.9, 7.6 Hz), 2.97 (dd, 1H, *J* =11.9, 4.9 Hz), 3.09 (quintet, 1H, *J* =6.6 Hz), 3.26 (dd, 1H, *J* =9.3, 3.5 Hz), 3.40 (s, 3H), 3.40-3.60 (m, 5H), 3.71 (m, 1H), 3.81 (m, 1H), 4.72 (d, 1H, *J*=3.6 Hz). ¹³C-NMR (CDCl₃) δ= 14.6, 23.1, 24.33, 24.37, 26.4, 26.5, 29.8, 29.9-30.1 (signals of linear C₁₄ chain CH₂), 30.8, 32.3, 33.1, 33.4, 51.7, 55.6, 60.3, 68.6, 71.9, 74.0, 75.6, 80.5, 81.2, 98.5. [α]²⁰_{D}: +18.4 (c 0.5, CHCl₃). (HRMS (FT-ICR): calcd for C₄₀H₈₀NO₅ [M+H]⁺: 654.6031, [M+Na]⁺: 676.5850; found: 654.6009; 676.5872.

### Step g) Methyl 6-deoxy-6-N,N',N''-dimethylcyclopentylammonium-2,3-di-O-tetradecyl-α-D-glucopyranoside (Id)

A solution of compound (**Ic**) (250 mg, 0.38 mmol), methyl iodide (50 µL, 0.76 mmol) and sodium carbonate (120 mg) in dry DMF (7 mL), was stirred under argon atmosphere at r.t. for 12 h. After this time the reaction was complete (TLC, 7:3 EtOAc/MeOH). The solvent was evaporated off in vacuo, the residue was dissolved in chloroform (15 mL) and washed with brine. The organic phase was dried over sodium sulphate, filtered and the solvent was evaporated off. Pure compound (**Id**) (241 mg, 93% yield) was obtained as a white powder. ¹H-NMR (CDCl₃) δ= 0.80 (bt, 6H, *J*= 6.8 Hz), 1.21 (m, 44H), 1.51 (m, 4H), 1.66 (m, 1 H), 1.80 (m, 2H), 2.15 (m, 1H), 3.13 (dd, 1H, *J* =9.8, 3.5 Hz), 3.21 (s, 3H), 3.23 (s, 3H), 3.32 (bt, 1H, *J*=9.2 Hz), 3.42 (s, 3H), 3.40-3.55 (m, 4H), 3.68 (bt, 1H, *J*=6.9 Hz), 3.91 (bs, 1H), 4.05 (bt, 1H, *J*=9.0 Hz), 4.13 (bt, 1H, *J*=8.5 Hz), 4.19 (d, 1H, *J*=14.1 Hz), 4.66 (d, 1H, *J*=3.5 Hz). ¹³C-NMR (CDCl₃) δ= 14.6, 23.1, 24.33, 24.37, 26.4, 26.5, 29.8, 29.9-30.1 (signals of linear C₁₄ chain CH₂), 30.8, 32.3, 50.5, 57.8, 66.1, 67.3, 71.7, 72.3, 74.0, 76.7, 79.7, 80.2, 99.6. [α]²⁰_{D}: +21.6 (c 0.5, CHCl₃). HRMS (FT-ICR): calcd for C₄₂H₈₅NO₅⁺ [M+H]⁺: 682.6344; found: 682.6354. Elemental analysis calcd, (%) for C₄₂H₈₄NO₅⁺: C 73.84, H 12.39, N 2.05; found: C 73.78, H 12.11, N 1.99.

### PHARMACOLOGY

### Materials and methods

### Dendritic cells, macrophages and culture medium

BMΦ and DC were derived from bone marrow cells and divided in two separate cultures. For DC preparation bone marrow cells were cultured in complete IMDM supplemented with 10% supernatant of GM-CSF-transduced B16 tumor cells [R. Soiffer, T. Lynch, M. Mihm, K. Jung, C. Rhuda, J. C. Schmollinger, F. S. Hodi, L. Liebster, P. Lam, S. Mentzer, S. Singer, K. K. Tanabe, A. B. Cosimi, R. Duda, A. Sober, A. Bhan, J. Daley, D. Neuberg, G. Parry, J. Rokovich, L. Richards, J. Drayer, A. Berns, S. Clift, L. K. Cohen, R. C. Mulligan, G. Dranoff, Proc. Natl. Acad. Sci. U S A. 1998, 95(22), 13141-13146]. Fresh medium was added every 2 d. After 7-10 d of culture, cells were analyzed for CD11c expression and used in assays when 90% were CD11c positive. For BMΦ preparation bone marrow cells were cultured in complete IMDM supplemented with 10% supernatant of M-CSF-transduced NIH3T3 cells. Also in this case, fresh medium was added every 2 d. After 7-10 d of culture, cells were analyzed for Mac1 expression and used in assays when at least 90% were Mac1 positive. D1 cells were cultured in complete IMDM supplemented with 10% supernatant of GM-CSF-transduced B16 tumor cells.

### Cell assays

D1 cells, BMDC and MΦ were plated in 48 well plates at a concentration of 200000 cells/well in 200µL of medium. One or two hours after plating they were treated with different amounts of compounds Ic and Id for 30 min and then stimulated with Lipid A from *Escherichia coli* (F583, Rd mutant Sigma, 0.5µM) for 18 h.

### HEK-293-hTLR-4A or -hTLR9A cell assay

Cells (InvivoGen, San Diego, CA, USA) were plated in 5 well plates at a concentration of 9.10⁶ cells/well in 5 mL of medium. Monosaccharide Id was then added to the medium and after 2 h lipid A from *Escherichia coli* or CpG (0.5 µM) was added to TLR-4 or TLR9 transfected cells, respectively. After 2 h the cells were collected for the measurement ofNFkB.

### Transcription factor NF-κB assay

Transcription factor analysis was performed with an ELISA kit (Active Motif, Rixensart, Belgium) that allowed for the detection of NF-kB activation by a combination of NF-kB-specific oligonucleotide binding and subsequent detection of the p65 subunit of NF-kB with a specific antibody, according to the manufacturer's instructions.

### Neuropathic pain

Male mice C57BL/6J were anesthetized with sodium pentobarbital (65 mg/kg, i.p.) and submitted to surgery to induce neuropathic pain. Briefly, the common sciatic nerve was exposed at the level of the mid thigh and, proximal to the sciatic nerves trifurcation, three ligatures were tied until a brief twitch was seen in the respective hindlimb. Sham animals (sciatic exposure without ligation) were used as controls. The animal pain response was monitored before surgery, on day 7 (24 h after the last administration). Compound (**Id**) was administered i.p. at 5 mg/kg once a day starting the day after the surgery. Heat hypersensitivity was tested according to the Hargreaves' procedure using the plantar test (Ugo Basile, Varese, Italy). Mechanical allodynia was measured using the Von Frey test.

### Cerebral ischemia on male Mongolian gerbils (Meriones unguiculatus)

After implanting EEG electrodes, the gerbils were divided into different groups on the basis of the assigned treatment. Their electroencephalogram (EEG) was recorded for 1 h a day, for three days, to determine the basal total and relative spectral power. The signals were recorded and processed for fast Fourier transform spectral analysis by means of PC software (PowerLab, AD Instruments, Pty, Ltd, Australia). EEG recordings were also made for seven days after ischemia. After basal EEG recordings, each gerbil was again lightly anaesthetised and 10-min. ischemia was induced by bilateral common carotid arteries occlusion. The ischemia was verified qualitatively on paper by the complete flattening of the EEG. A group of animals (sham-operated) underwent the same surgical procedure except that the carotid arteries were not clamped. Compound **Id** was i.p. administered to the animals at the dose of 10 mg/kg 30 minutes before ischemia induction.

### Experiments and results

Three representative compounds of the invention, compounds **Ia, Ic** and **Id,** were tested for their ability to interfere with lipid A-induced dendritic cells (DC) and macrophages (MΦ) activation. These two classes of leukocytes are directly involved in boosting and controlling inflammatory responses and, upon interaction with microbial products such as LPS, they produce large amounts of TNFα, one of the main mediators of inflammation and septic shock. In this context, LPS activation of TLR-4 represents one of the most potent stimuli for DC priming.

Given the pivotal role of early-activated DC and MΦ in directing inflammatory reactions, compounds **Ia, Ic** and **Id** were tested for their ability to antagonize the stimulatory activity of lipid A on bone marrow derived macrophages (BMΦ) and DC (BMDC) by interfering with their capacity to induce TNFα production. As source of DC, D1 cells, a long term growth factor (GM-CSF)-dependent DC line [C. Winzler et al. J. Exp. Med. 1997, 185, 317-327] were also used. BMΦ, BMDC and D1 cells were stimulated with lipid A (0.5 µM) after a pre-exposure to compounds **Ia, Ic** and **Id** and the amount of TNFα released in the supernatant was measured by ELISA. As shown in Figure 1, monosaccharides **Ic** and **Id** interfered with lipid A's action in a dose-dependent manner. Compound **Id** showed a lower activity while monosaccharide **10,** lacking the tetrahydrofuranyl ring, was totally inactive (data not shown). In general, compounds **Ia, Ic** and **Id** did not show any direct inflammatory function, as they were not capable of stimulating cytokines production. Compound **Id** was more potent than **Ic** that was in turn a better inhibitor than **Ia.** Moreover, compound **Id** showed the highest solubility in the aqueous media used in the tests.

Compound **Id** was then studied in more detail by monitoring the production of a second inflammatory cytokine, IL-1β. IL-1β and TNFα synthesis were inhibited in BMΦ and D1 cells and the effect was proportional to **Id** concentration. In order to evaluate the selectivity for TLR-4, TNFα production was investigated in the presence of compound **Id** in response to stimulation through the CpG motif of bacterial DNA recognized by TLR9 [H. Hemmi et al., Nature 2000, 408, 740-745] and tripalmitoyl cysteine (Pam₃Cys-SK₄), which is specific for TLR2 [S. Agrawal et al., J. Immunol. 2003, 171(10), 4984-4989]. TNFα production was not inhibited by **Id** in both TLR9 and TLR2-mediated inflammatory cascades (Figure 2), indicating a relevant level of selectivity.

The cytotoxic potential of compounds **Ia, Ib** and **Id** was also investigated. The compounds of the invention contain a polar part (the sugar moiety) linked to lipophilic chains, and can therefore act as detergent and kill cells by generating pores into the plasma membranes. The toxicity of compounds **Ia, Ib** and **Id** was investigated with the PI test and the apoptotic potential was investigated with the annexin V test. Neither DC nor MΦ showed an appreciable percent of dead cells after 48 h incubation with **Id** at concentrations ranging from 10 to 100 µM (Figure 3).

A further direct evidence that compound Id selectively exerts its action on the TLR-4 receptor was obtained with experiments on TLR-4- and TLR9-transfected HEK 293 cell system [J.C. Chow, J. Biol. Chem. 1999, 274(16), 10689-10692]. In these cells TLR-dependent NF-kB activation can be easily measured after stimulation. The activation of TLR-4 signalling pathway leads to NF-kB nuclear translocation and inflammatory cytokine production. TLR-4- and TLR9-transfected HEK 293 were incubated respectively with lipid A (0.5µM) or CpG (0.5 µM), after a pre-exposure to compound **Id** and the activation of NF-kB was measured 2 h later. As shown in Figure 4, monosaccharide **Id** was able to significantly counteract the effect of lipid A in TLR-4-transfected cells, while it was inactive at the maximal dose of 50 µM in contrasting CpG effect on TLR9 HEK 293.

The repeated administration of compound **Id** to neuropathic mice once a day for one week, starting the day after the lesion of the sciatic nerve induced a significant relief of both signs of neuropathic pain: thermal hyperalgesia (reduced withdrawal latency to thermal stimulus) and mechanical allodynia (pain perception after application of a usually innocuous mechanical stimulus), as shown in Figure 5.

Compound **Id** was also able to significantly antagonize the ischemia-induced EEG flattening starting from 1 hour after recirculation. A quantitative EEG analysis of gerbils treated with compound **Id** is shown in Figure 6. Two-way ANOVA revealed a significant dose, time and interaction effect between groups evaluated during ischemia and 1 hour, 1 day, 3 days and 7 days after recirculation. The vehicle group had a significant decrease in EEG power at all tested intervals.

### In vivo activity in mice

The anti-inflammatory activity of compound **Ia** was evaluated with the carrageenan edema test on C57BL/6J (Harlan, Italy) male rats aged 9-weeks, maintained under standard conditions of temperature, humidity and light/dark cycle and allowed to acclimate for at least one week before the test. The mice were anesthetized with sodium pentobarbital (60 mg/kg, i.p.) and acute inflammation was induced by intraplantar administration of 20 µl of λ-carrageenan (2% physiological solution). Control animals received an intraplantar administration of physiological solution. Compound **Ia** (dissolved in 10% ethanol in physiological solution) was administered intraperitoneally 30 minutes before carrageenan at different doses (3, 10 and 30 mg/kg, 100 µl/10g body weight). Control animals received an analogous administration of vehicle. A group of animals received indomethacin (5 mg/kg, i.p.) as known antiinflammatory for an efficacy comparison.

The paw volume was measured with a plethysmometer before and 2 hours after carrageenan administration. The difference between the volume of the ipsilateral and the contralateral paw represents the antinflammatory oedema expressed as µl. The results are reported in figure 7.

Nitric oxide production *ex vivo* in paws homogenate was measured as meaningful marker of inflammation, through a fluorimeric procedure based on the determination of nitrites/nitrates level, which are the final products of NO metabolism [Misko et al., Anal. Biochem. 1993, 214, 11-16]. The results are reported in figure 8.

## Claims

1. Compounds of general formula (**I**) 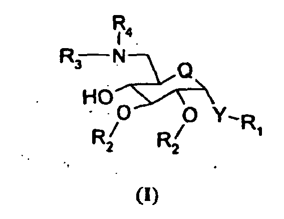 wherein:
Q represents oxygen;
Y represents oxygen;
R₁ is selected from hydrogen, C₁-C₁₀ alkyl and phenyl;
R₂ are both C₁₄ alkyl chains;
R₃ is tetrahydrofuran-1-yl or cyclopentyl;
R₄ is selected from hydrogen, C₁-C₁₀ alkyl or a R₄'X group wherein
R₄' is C₁-C₁₀ alkyl and X is an oxygen or sulphur atom;
and physiologically acceptable acid salts or quaternary ammonium salts thereof.

2. A compound selected from:
methyl 6-deoxy-6-[N-(1'-tetrahydrofuranyl)-N'-methoxyamino)-2,3-di-O-tetradecyl-α-D-glucopyranoside **(Ia)** 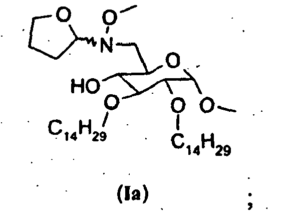
methyl 6-deoxy-6-[N-(cyclopentyl)-N'-methoxyamino)-2,3-di-O-tetradecyl-α-D-glucopyranoside **(Ib)** 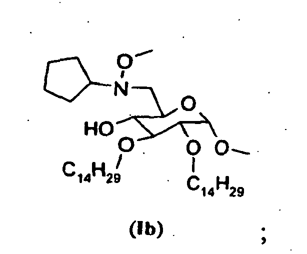
methyl 6-deoxy-6-cyclopentylamino-2,3-di-*O*-tetradecyl-α-D-glucopyranoside **(Ic)** 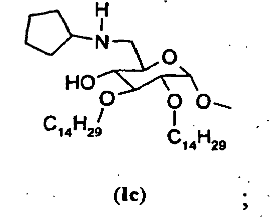
methyl 6-deoxy-6-*N,N',N"*-dimethylcyclopentylammonium-2,3-di-*O*-tetradecyl-α-D-glucopyranoside **(Id)**

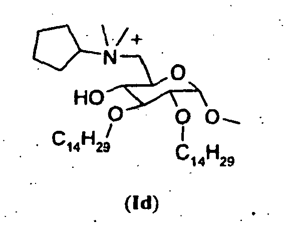

3. Compounds of any one of claims 1 or 2 for use as medicaments.

4. Use of the compounds of any one of claims 1 to 2 for the preparation of pharmaceutical compositions for the treatment of inflammatory conditions.

5. Use of the compounds of any one of claims 1 to 2 for the preparation of analgesic pharmaceutical compositions.

6. Use of the compounds of any one of claims 1 to 2 for the preparation of pharmaceutical compositions for the treatment of cerebral ischemia.

7. Pharmaceutical compositions containing a compound of any one of claims 1 to 2 in admixture with suitable excipients and/or vehicles.

## Patentansprüche

1. Verbindungen der allgemeinen Formel **(I)** 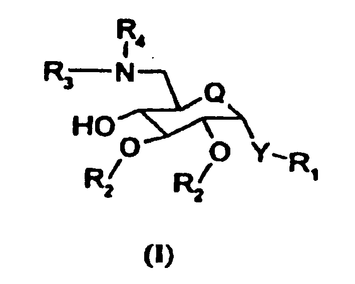 worin:
Q Sauerstoff darstellt;
Y Sauerstoff darstellt;
R₁ ausgewählt ist aus Wasserstoff, C₁-C₁₀-Alkyl und Phenyl;
R₂ jeweils C₁₄-Alkyl-Ketten sind;
R₃ Tetrahydofuran-1-yl oder Cyclopentyl ist;
R₄ ausgewählt ist aus Wasserstoff, C₁-C₁₀-Alkyl oder einer R₄'X-Gruppe, wobei R₄' ein C₁-C₁₀-Alkyl und X ein Sauerstoff- oder Schwefelatom ist;
und Salze physiologisch annehmbarer Säuren oder quaternäre Ammoniumsalze davon.

2. Verbindung ausgewählt aus:
Methyl-6-deoxy-6-[N-(1'-tetrahydrofuranyl)-N'-methoxyamino)-2,3-di-O-tetradecyl-α-D-glucopyranosid **(Ia)** 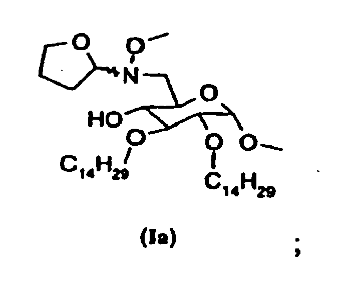
Methyl-6-deoxy-6-[N-(cyclopentyl)-N'-methoxyamino)-2,3-di-O-tetradecyl-α-D-glucopyranosid **(Ib)** 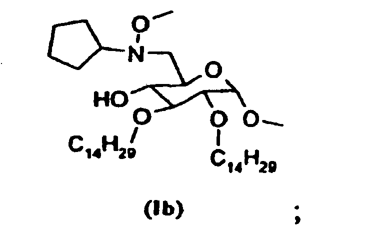
Methyl-6-deoxy-6-cyclopentylamino-2,3-di-O-tetradecyl-α-D-glucopyranosid **(Ic)** 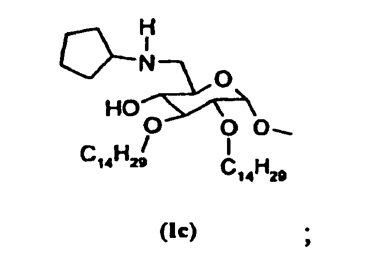
Methyl-6-deoxy-6-N,N',N"-dimethylcyclopentylammonium-2,3-di-O-tetradecyl-α-D-glucopyranosid **(Id)**

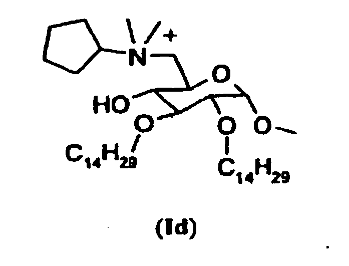

3. Verbindungen nach einem der Ansprüche 1 oder 2 zur Verwendung als Medikamente.

4. Verwendung der Verbindungen nach einem der Ansprüche 1 bis 2 für die Herstellung von pharmazeutischen Zusammensetzungen für die Behandlung entzündlicher Zustände.

5. Verwendung der Verbindungen nach einem der Ansprüche 1 bis 2 für die Herstellung von analgetischen pharmazeutischen Zusammensetzungen.

6. Verwendung der Verbindungen nach einem der Ansprüche 1 bis 2 für die Herstellung von pharmazeutischen Zusammensetzungen für die Behandlung von zerebraler Ischämie.

7. Pharmazeutische Zusammensetzungen, die eine Verbindung nach einem der Ansprüche 1 bis 2 in Beimischung mit geeigneten Hilfsstoffen und/oder Vehikeln enthalten.

## Revendications

1. Composés de formule générale (I) 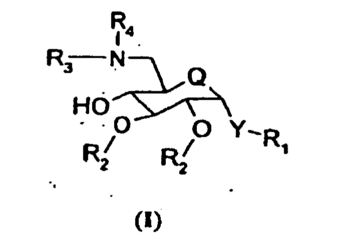 dans laquelle
Q représente un oxygène ;
Y représente un oxygène ;
R₁ est choisi parmi un hydrogène, un alkyle en C₁-C₁₀ et un phényle ;
R₂ sont tous deux des chaines alkyles en C₁₄ ;
R₃ est un tétrahydrofuran-1-yle ou un cyclopentyle ;
R₄ est choisi parmi un hydrogène, un alkyle en C₁-C₁₀ ou un groupe R₄'X dans lequel
R₄' est un alkyle en C₁-C₁₀ et X est un atome d'oxygène ou de soufre ;
et des sels acides ou des sels d'ammonium quaternaire de ceux-ci physiologiquement acceptables.

2. Un composé choisi parmi :
le méthyl 6-déoxy-6-[N-(1'-tétrahydrofuranyl)-N'-méthoxyamino)-2,3-di-O-tétradécyl-α-D-glucopyranoside (Ia) 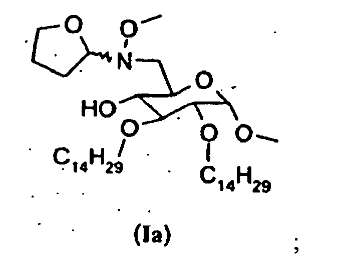
le méthyl 6-déoxy-6-[N-(cyclopentyl)-N'-méthoxyamino)-2,3-di-O-tétradécyl-α-D-glucopyranoside (Ib) 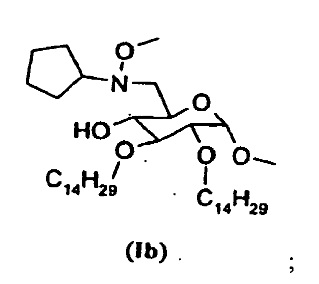
le méthyl 6-déoxy-6-cyclopentylamino-2,3-di-*O*-tétradécyl-α-D-glucopyranoside (Ic) 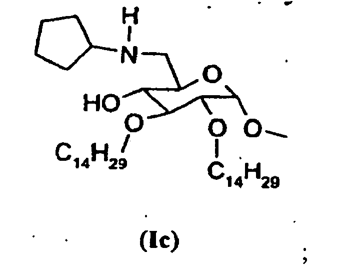
le méthyl 6-déoxy-6-*N, N', N"*-diméthylcyclopentylammonium-2,3-di-*O*-tétradécyl-α-D-glucopyranoside (Id)

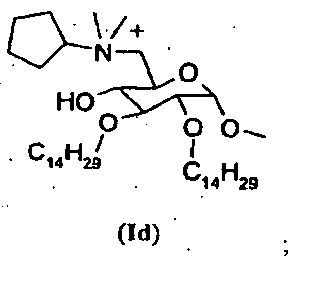

3. Composés de l'une quelconque des revendications 1 ou 2 pour une utilisation en tant que médicaments.

4. Utilisation des composés de l'une quelconque des revendications 1 à 2 pour la préparation de compositions pharmaceutiques pour le traitement de conditions inflammatoires.

5. Utilisation des composés de l'une quelconque des revendications 1 à 2 pour la préparation de compositions pharmaceutiques analgésiques.

6. Utilisation des composés selon l'une quelconque des revendications 1 à 2 pour la préparation de compositions pharmaceutiques pour le traitement de l'ischémie cérébrale.

7. Compositions pharmaceutiques contenant un composé de l'une quelconque des revendications 1 à 2 en mélange avec des excipients et/ou des véhicules appropriés.
